# FASCICULE DE BREVET EUROPEEN

(11) **EP 1 755 582 B1**
(45) Date de publication et mention de la délivrance du brevet: **20.06.2012**
(21) Numéro de dépôt: 05763719.1
(22) Date de dépôt: 29.04.2005
(51) Int. Cl.: A61K 31/198, A61K 31/40, A61P 9/10

(54) **Compositions pharmaceutiques pour la prevention et le traitement de l'atherosclerose**
Pharmazeutische Zusammensetzungen zur Vorbeugung und Behandlung von Atherosklerose
Pharmaceutical compositions for the prevention and treatment of atherosclerosis

(30) Priorité: 29.04.2004 FR 0404569
(43) Date de publication de la demande: 28.02.2007
(73) Titulaire: Université Paris Descartes, 75270 Paris Cedex 06 (FR); Assistance Publique - Hopitaux De Paris, 75100 Paris RP (FR)
(72) Inventeur: COUDERC, Rémy, F-92340 Bourg La Reine (FR); RASMUSEN, Carole, F-95250 Beauchamp (FR); MARTIN, Chantal, F-75008 Paris (FR); TRICOTTET, Viviane, F-77190 Dammarie Les Lys (FR); CYNOBER, Luc, F-92390 Sceaux (FR); MOINARD, Christophe, F-92340 Bourg La Reine (FR)
(74) Mandataire: Grosset-Fournier, Chantal Catherine
(86) Numéro de dépôt international: PCT/FR2005/001083
(87) Numéro de publication internationale: WO 2005/115371

(56) Documents cités:
- EP-A- 0 104 041
- EP-A- 0 312 157
- WO-A-03/082816
- US-A- 4 650 661
- US-A- 4 749 687
- US-A1- 2003 114 515

## Description

La présente invention concerne une nouvelle composition pharmaceutique et son utilisation notamment dans le cadre de la lutte contre l'athérosclérose.

L'athérosclérose est la première cause de mortalité dans les pays industrialisés, avec plus de 20% des décès (Murray JL, Lopez AD. Mortality by cause for eight regions of the world : global burden of disease study. Lancet 1997 ; 349 : 1269-76). Selon l'OMS (1954), l'athérosclérose est une association variable de remaniements de l'intima des artères de gros et moyen calibre consistant en la formation d'une plaque d'athérome, c'est à dire une accumulation locale de lipides, de glucides complexes, de sang et de produits sanguins, de tissu fibreux et de dépôts calcaires ; le tout s'accompagne de modifications de la média. Cette pathologie ne se manifeste qu'au bout de plusieurs années d'évolution infra-clinique. Sa prévention, plus encore que son traitement, reste donc capitale.

La formation de la plaque est une succession de cinq étapes (Tedgui A, Mallat Z. Formation de la plaque d'athérosclérose. Rev Prat 1999 ; 49 : 2081-2086) :
- accumulation de LDL (Low Density Lipoprotein) dans l'intima,
- oxydation des LDL (LDLox) dans la paroi par le contact avec des cellules endothéliales, des cellules musculaires lisses (CML) ou des macrophages,
- recrutement de monocytes circulants grâce à l'induction de molécules d'adhésion, telles que VCAM1 ou ICAM1, provoquée par les LDL oxydées,
- captage des LDLox par les macrophages grâce aux récepteurs « scavenger » ; ainsi le cholestérol s'accumule dans les macrophages,
- formation d'une chape fibromusculaire stabilisante, constituée de cellules musculaires lisses et de protéines de la matrice extracellulaire.

La L-Arginine, acide aminé basique présent dans les protéines végétales et animales, est indispensable à la synthèse des protéines tissulaires. Dans différents travaux chez l'animal, lors d'une supplémentation du régime en L-Arginine (à 2% dans l'eau de boisson, dose permettant de doubler la concentration plasmatique d'arginine), il semble que celle-ci tend à avoir un effet antiathérogène. Ce traitement semble également avoir un effet sur la réactivité vasculaire. Böger & al (Bôger RH, Bode-Böger SM, Brandes RP, Phivthong-ngam L, Böhme M, Nafe R, et al. Dietary L-Arginine reduces the progression of atherosclerosis in cholesterol-fed rabbits. Circulation 1997 ; 96 : 1282-1290), ainsi que Behr-Roussel & al (Behr-Roussel D, Rupin A, Simonet S, Bonhomme E, Coumailleau S, Cordi A, et al. Effect of chronic treatment with the inducible nitric oxide synthase inhibitor N-iminoethyl-L-lysine or with L-arginine on progression of coronary and aortic atherosclerosis in hypercholesterolemic rabbits. Circulation 2000 ; 102 : 1033-1038) mettent en évidence une stabilisation de l'aire de la plaque athéromateuse avec un régime enrichi pendant 12 à 16 semaines.

Les statines sont de puissants inhibiteurs de la synthèse du cholestérol. Leur indication principale est la réduction du LDL-cholestérol plasmatique en prévention primaire ou secondaire des accidents ischémiques cardiaques (Vaughan CJ, Murphy MB, Buckley BM. Statins do more than just lower cholesterol. Lancet 1996 ; 348 : 1079-1082).

Les statines inhibent l'HMG-CoA réductase par compétition réversible avec le substrat HMG-CoA, pour le site actif de l'enzyme. Elles entraînent donc une diminution de la synthèse du mévalonate et de ses dérivés parmi lesquels le cholestérol. De ce fait, les statines sont indiquées pour le traitement de l'athérosclérose.

Toutefois, à l'heure actuelle, aucun des traitements cités ne permet de prévenir et/ou de traiter totalement l'athérosclérose.

Un objectif de l'invention est donc de fournir un mode de prévention et/ou de traitement plus efficace de l'athérosclérose.

La présente invention découle notamment de la mise en évidence d'un effet synergique inattendu de l'association d'une statine et de L-Arginine dans le cadre de la prévention et/ou du traitement de l'athérosclérose.

Ainsi, la présente invention concerne l'utilisation,
- d'au moins une statine, l'atovarstatine ou d'un sel pharmaceutiquement acceptable de celle-ci, en association avec
- au moins un composé de formule (I) suivante : 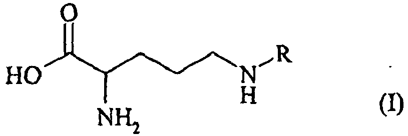 dans laquelle R représente, 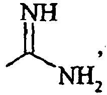 le composé de formule (1) correspondant à l'arginine ou un sel pharmaceutiquement acceptable de celui-ci,
pour la préparation d'un médicament destiné à la prévention ou au traitement de l'athérosclérose, notamment de l'athérosclérose primaire ou secondaire,

L'hypertension, le diabète et les maladies neurodégénératives, notamment la maladie d'Alzheimer, présentent une composante vasculaire liée à une dysfonction endothéliale pouvant être compensée par l'association synergique d'un composé de formule (I) et de statine, notamment par l'association synergique arginine-statine, selon l'invention.

L'expression « en association » signifie que la statine et le composé de formule (I) sont tous deux présents de manière indépendante dans les médicaments selon l'invention, où ils ne sont en aucun cas liés entre eux, que ce soit par le biais de liaisons faibles, telles que des interactions électrostatiques, pour former un sel par exemple, ou par le biais de liaisons fortes, telles que des liaisons covalentes.

Comme on l'entend ici, le terme « statine » désigne un composé appartenant à la classe des inhibiteurs de HMG-CoA réductase. En particulier, le terme statine ne correspond en aucun cas à un dérivé d'acide aminé issu de la pepstatine et ayant des propriétés d'inhibition de la rénine.

Par ailleurs, avantageusement, le composé de formule générale (I), notamment l'arginine, est utilisé comme adjuvant pour augmenter les effets des statines dans le cadre de la préparation de médicaments destinés à la prévention ou au traitement de l'athérosclérose, notamment de l'athérosclérose primaire ou secondaire, de l'hypertension, du diabète, ou des maladies neurodégénératives, notamment la maladie d'Alzheimer.

Selon un mode de réalisation préféré de l'invention, la statine est choisie parmi le groupe comprenant :
l'atorvastatine,

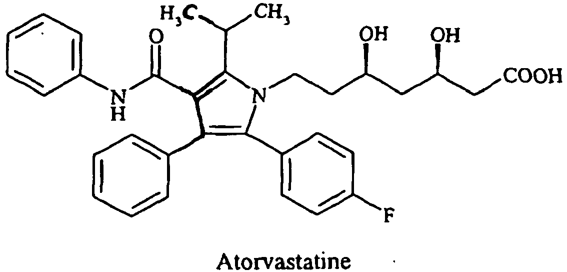

Selon un mode de réalisation particulièrement préféré de l'invention, la statine est l'atorvastatine.

Selon un autre mode de réalisation préféré de l'invention, le composé de formule (I) correspond à l'isomère L.

Selon encore un autre mode de réalisation préféré de l'invention, R représente 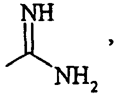 le composé de formule (I) correspondant alors à l'arginine, notamment à la L-Arginine.

L'invention concerne plus particulièrement l'utilisation telle que définie ci-dessus, d'une statine, notamment d'atorvastatine, en une quantité convenant pour l'administration à un individu d'une dose d'environ 5 mg/j à environ 80 mg/j.

L'invention concerne également plus particulièrement l'utilisation telle que définie ci-dessus, d'un composé de formule (I), notamment de L-Arginine, en une quantité convenant pour l'administration à un individu d'une dose d'1 g/jour à 30 g/jour, notamment d'5 g/jour à 30 g/jour.

De manière avantageuse les doses de L-Arginine inférieures à 5 g/j conviennent particulièrement aux enfants.

L'invention concerne également tout particulièrement l'utilisation telle que définie ci-dessus, de L-Arginine, en une quantité convenant pour l'administration à un individu d'une dose d'au moins 10 g/jour.

Dans un mode de réalisation particulier, l'invention concerne l'utilisation, telle que définie ci-dessus, de L-Arginine en une quantité convenant pour l'administration à un individu d'une dose de 0,15 g/kg/jour, c'est-à-dire une dose de 0,15 g/jour de L-Arginine par kg de poids corporel de l'individu en question.

Selon un autre mode de réalisation particulier de l'utilisation telle que définie ci-dessus, le médicament convient pour l'administration à un individu d'une dose unitaire de 5 mg à 80 mg de statine et de 5 g à 30 g, notamment 10 g, de L-Arginine.

La présente invention concerne également des produits contenant
- au moins une statine l'atorvastatine ou un sel pharmaceutiquement acceptable de celle-ci, et
- au moins un composé de formule (I) suivante : 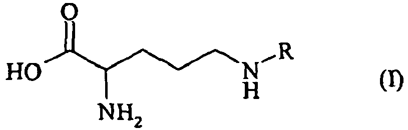 dans laquelle R représente, 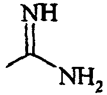 ou un sel pharmaceutiquement acceptable de celui-ci,
comme produits de combinaison pour une utilisation simultanée, séparée ou étalée dans le temps dans le cadre du traitement de l'athérosclérose, notamment de l'athérosclérose primaire ou secondaire,

L'expression « produits de combinaison » signifie que la statine et le composé de formule (I) sont tous deux présents de manière indépendante dans les produits selon l'invention, où ils ne sont en aucun cas liés entre eux, que ce soit par le biais de liaisons faibles, telles que des interactions électrostatiques, pour former un sel par exemple, ou par le biais de liaisons fortes, telles que des liaisons covalentes.

Selon un mode de réalisation préféré de l'invention, la statine est l'atorvastatine.

Selon un mode de réalisation particulièrement préféré, la statine est l'atorvastatine.

Selon un autre mode de réalisation préféré de l'invention, le composé de formule (I) correspond à l'isomère L.

Selon encore un autre mode de réalisation préféré de l'invention, R représente 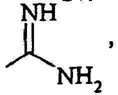 le composé de formule (I) correspondant alors à l'arginine, notamment à la L-Arginine.

Selon un mode de réalisation particulièrement préféré de l'invention, lesdits produits conviennent pour l'administration à un individu d'une dose de 5 mg/j à 80 mg/j de statine, notamment d'atorvastatine.

Selon un autre mode de réalisation particulièrement préféré de l'invention, lesdits produits conviennent pour l'administration à un individu d'une dose de 1 g/jour à 30 g/jour, notamment de 5 g/jour à 30 g/jour, d'un composé de formule (I), notamment de L-Arginine.

De manière avantageuse les doses de L-Arginine inférieures à 5 g/j conviennent particulièrement aux enfants.

Selon un autre mode de réalisation de l'invention particulièrement préféré, lesdits produits conviennent pour l'administration à un individu d'une dose de L-Arginine d'au moins environ 10 g/jour.

Dans un mode de réalisation particulier, lesdits produits conviennent pour l'administration à un individu d'une dose de L-Arginine de 0,15 g/kg/jour.

Selon un autre mode de réalisation particulier des produits tels que définis ci-dessus, les produits conviennent pour l'administration à un individu d'une dose unitaire de 5 mg à 80 mg de statine et de 5 g à 30 g, notamment 10 g, de L-Arginine.

La présente invention concerne également une composition pharmaceutique comprenant à titre de substance active:
- au moins une statine l'atorvastatine ou un sel pharmaceutiquement acceptable de celle-ci, et
- au moins un composé de formule (I) suivante : 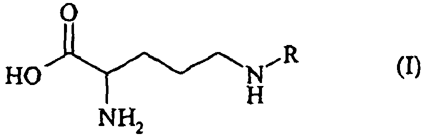 dans laquelle R représente, 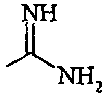 ou un sel pharmaceutiquement acceptable de celui-ci,
en association avec un véhicule pharmaceutiquement acceptable.

Ces trois acides aminés possèdent un métabolisme commun dans l'organisme.

Comme on l'entend ici, l'atorvastatine et le composé de formule (I) sont tous deux présents de manière indépendante dans les compositions pharmaceutiques selon l'invention, où ils ne sont en aucun cas liés entre eux, que ce soit par le biais de liaisons faibles, telles que des interactions électrostatiques, pour former un sel par exemple, ou par le biais de liaisons fortes, telles que des liaisons covalentes.

Selon un mode de réalisation préféré de l'invention, la statine est l'atorvastatine.

Selon un mode de réalisation particulièrement préféré de l'invention, la statine est l'atorvastatine.

Selon un autre mode de réalisation préféré de l'invention, le composé de formule (I) correspond à l'isomère L.

Les acides aminés naturels retrouvés dans l'organisme sont essentiellement de type L. selon encore un autre mode de réalisation préféré de l'invention, R représente 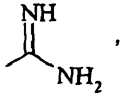 le composé de formule (I) correspondant alors à l'arginine, notamment à la L-Arginine.

Selon un autre mode de réalisation préféré de l'invention, la composition pharmaceutique selon l'invention convient pour l'administration à un individu d'une dose de 5 mg/j à 80 mg/j de statine, notamment d'atorvastatine.

Selon encore un autre mode de réalisation préféré de l'invention, la composition pharmaceutique selon l'invention convient pour l'administration à un individu d'une dose de g/jour à 30 g/jour, notamment de 5 g/jour à 30 g/jour, d'un composé de formule (I), notamment de L-Arginine.

De manière avantageuse les doses de L-Arginine inférieures à 5 g/jour conviennent particulièrement aux enfants.

Selon un mode de réalisation particulièrement préféré, la composition pharmaceutique selon l'invention convient pour l'administration à un individu d'une dose de L-Arginine d'au moins 10 g/jour.

Dans un mode de réalisation particulier, la composition pharmaceutique telle que définie ci-dessus convient pour l'administration à un individu d'une dose de L-Arginine de 0,15 g/kg/jour.

Selon un autre mode de réalisation particulier des compositions pharmaceutiques telles que définies ci-dessus, les compositions pharmaceutiques conviennent pour l'administration à un individu d'une dose unitaire de 5 mg à 80 mg de statine et de 5 g à 30 g, notamment 10 g, de L-Arginine.

Selon un autre mode de réalisation préféré de l'invention, la composition pharmaceutique selon l'invention convient pour une administration par voie orale.

Selon encore un autre mode de réalisation préféré de l'invention, la composition pharmaceutique selon l'invention est caractérisée en ce qu'elle se présente sous forme de poudre à diluer, de cachet, de sachet, de comprimé, de gélule, ou sous tout autre forme galénique acceptable.

### DESCRIPTION DES FIGURES

**Figure 1**

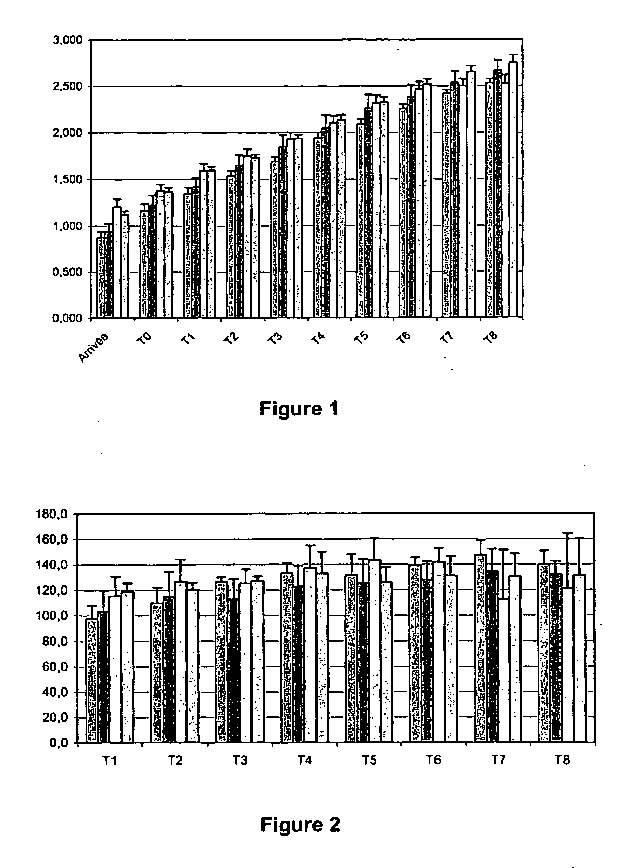

   La figure 1 représente l'évolution du poids des animaux (en kilogrammes, axe des ordonnées) en fonction du temps (axe des abscisses) depuis leur arrivée, puis du début (T0) à la fin du traitement (semaines T1 à T8) pour le groupe témoin (premier histogramme), le groupe arginine (deuxième histogramme), le groupe statine (troisième histogramme) et le groupe statine + arginine (quatrième histogramme).
**Figure 2**
   La figure 2 représente la prise alimentaire moyenne des animaux (en grammes/jours, axe des ordonnées) en fonction du temps (axe des abscisses) du début du traitement (T0) à la fin du traitement (semaines T1 à T8) pour le groupe témoin (premier histogramme), le groupe arginine (deuxième histogramme), le groupe statine (troisième histogramme) et le groupe statine + arginine (quatrième histogramme).
**Figure 3**

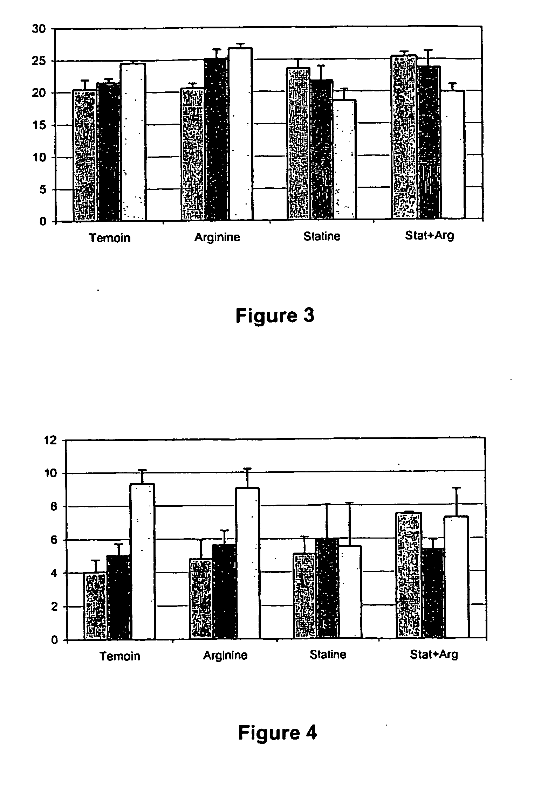

   La figure 3 représente la cholestérolémie moyenne (en mmol/l, axe des ordonnées) pour le groupe témoin, le groupe arginine, le groupe statine et le groupe statine + arginine, aux temps (axe des abscisses) T0 (premier histogramme), T4 (deuxième histogramme), T8 (sacrifice) (troisième histogramme).
**Figure 4**
   La figure 4 représente la triglycéridémie moyenne (en mmol/l, axe des ordonnées) pour le groupe témoin, le groupe arginine, le groupe statine et le groupe statine + arginine, aux temps (axe des abscisses) T0 (premier histogramme), T4 (deuxième histogramme), T8 (sacrifice) (troisième histogramme).
**Figure 5**

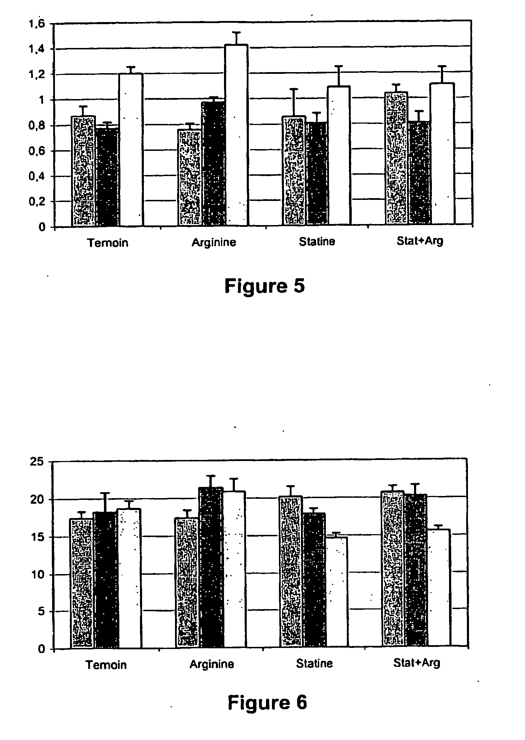

   La figure 5 représente la concentration plasmatique moyenne des HDL (en mmol/l, axe des ordonnées) pour le groupe témoin, le groupe arginine, le groupe statine et le groupe statine + arginine, aux temps (axe des abscisses) T0 (premier histogramme), T4 (deuxième histogramme), T8 (sacrifice) (troisième histogramme).
**Figure 6**
   La figure 6 représente la concentration plasmatique moyenne des LDL calculée d'après la formule de Frieldwald (en mmol/l, axe des ordonnées) pour le groupe témoin, le groupe arginine, le groupe statine et le groupe statine + arginine, aux temps (axe des abscisses) T0 (premier histogramme), T4 (deuxième histogramme), T8 (sacrifice) (troisième histogramme).
**Figure 7**

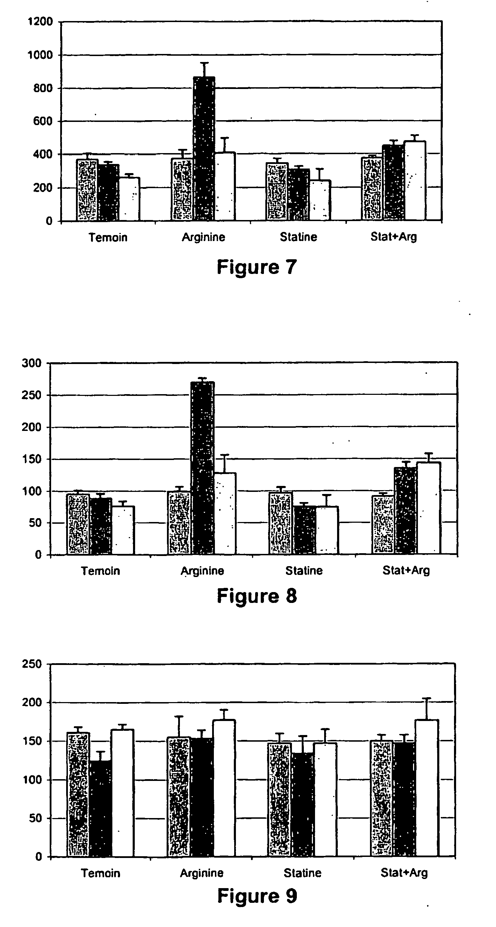

   La figure 7 représente la concentration plasmatique moyenne de l'arginine (en µmol/l, axe des ordonnées) pour le groupe témoin, le groupe arginine, le groupe statine et le groupe statine + arginine, aux temps (axe des abscisses) T0 (premier histogramme), T4 (deuxième histogramme), T8 (sacrifice) (troisième histogramme).
**Figure 8**
   La figure 8 représente la concentration plasmatique moyenne de l'ornithine (en µmol/l, axe des ordonnées) pour le groupe témoin, le groupe arginine, le groupe statine et le groupe statine + arginine, aux temps (axe des abscisses) T0 (premier histogramme), T4 (deuxième histogramme), T8 (sacrifice) (troisième histogramme).
**Figure 9**
   La figure 9 représente la concentration plasmatique de la citrulline (en µmol/l, axe des ordonnées) pour le groupe témoin, le groupe arginine, le groupe statine et le groupe statine + arginine, aux temps (axe des abscisses) T0 (premier histogramme), T4 (deuxième histogramme), T8 (sacrifice) (troisième histogramme).
**Figure 10**

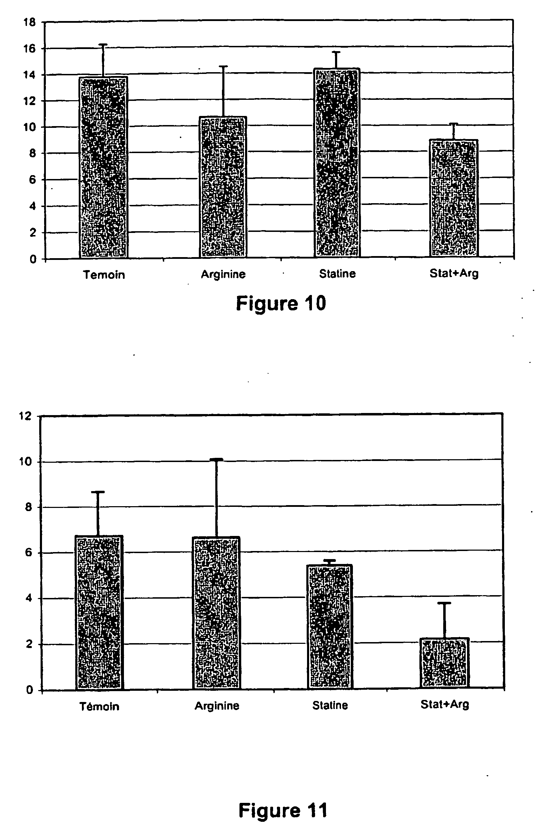

   La figure 10 représente le pourcentage de la surface des lésions (axe des ordonnées) dans les différents groupes (axe des abscisses) au moment du sacrifice (T8).
**Figure 11**
   La figure 11 représente le pourcentage de la surface des lésions (axe des ordonnées) au niveau distal de l'aorte dans les différents groupes (axe des abscisses) au moment du sacrifice (T8).

### EXEMPLE

### Matériels et Méthodes

### 1. Animaux et protocole expérimental

Vingt-quatre lapins Watanabe (Centre de production animale, Olivet, France) hyperlipidémiques par déficit héréditaire en récepteur des LDL (LDL-R) homozygotes, âgés de six semaines, sont utilisés. Chaque animal est placé dans une cage individuelle et reçoit une ration alimentaire de 200g à 250g par jour. Après 7 jours d'acclimatation à l'animalerie, quatre groupes de six lapins sont formés :
- un groupe témoin reçoit un régime normal pour lapin contenant 16 % de protéines, 3,2 % de lipides et 49,3 % de glucides (EXT C15, Dietex, St Gratien, France),
- les lapins du deuxième groupe, dit groupe « arginine », reçoivent un régime enrichi en L-Arginine de manière à doubler leur concentration plasmatique en cet acide aminé. La L-Arginine (Sigma, St Quentin Fallavier, France) a été mélangée à la nourriture, à raison de 15g/kg de nourriture réalisant un apport de 1g d'arginine/kg de poids vif/j,
- un troisième groupe, dit groupe « statine », reçoit un régime avec une statine (Atorvastatine, Tabor® 10mg, Godeche-Pfizer, Friburg, Allemagne) dans l'eau de boisson, à raison de 2,5mg/kg/j (Maeso R, Aragoncillo P & al Effect of Atorvastatin on endothelium-dependant constrictor factors in dyslipidemic rabbits. Gen Pharma. 2000 ; 34 : 263-272),
- et enfin un quatrième groupe, dit « groupe statine/arginine », reçoit un régime enrichi en arginine et un traitement par l'atorvastatine, également à 2,5mg/kg/j.

L'apport de L-Arginine dans le régime enrichi est réalisé de façon à correspondre à un apport d'environ 0,15 g/kg/j de L-Arginine chez l'homme.

La prise alimentaire est mesurée quotidiennement et les animaux sont pesés une fois par semaine. Des échantillons de plasma sont prélevés tous les quinze jours à partir de la veine marginale de l'oreille. Le sang, recueilli sur tubes héparinés, est centrifugé (4°C, 2000g, 15min). Une partie du plasma est déprotéinisée par une solution d'acide sulfosalicylique (30%) puis répartie en fractions aliquotes en vue du dosage des acides aminés. Le reste du plasma est aliquoté en plusieurs fractions d'environ 200 µl, et conservé à -80°C.

A l'issue de huit semaines, un prélèvement de sérum (environ 1mL) est effectué puis les animaux sont anesthésiés par une injection de pentobarbital hépariné, et enfin sacrifiés.

L'ensemble de l'étude est réalisé selon la réglementation en vigueur concernant l'expérimentation animale.

### 2. Analyses biochimiques

Le *bilan lipidique,* c'est-à-dire, cholestérol, triglycérides, cholestérol des HDL (High Density Lipoproteins) et des LDL (Low Density Lipoproteins), est effectué sur Hitachi 911 par les méthodes usuelles. Le cholestérol est dosé selon un test colorimétrique enzymatique à l'aide d'une cholestérol-estérase et d'une cholestérol-oxydase, suivi d'une réaction avec une peroxydase. Celle-ci réagit avec l'eau oxygénée et libère un dérivé rouge dont l'intensité de coloration (mesurée entre 550 et 700 nm) est directement proportionnelle à la concentration de cholestérol. Le dosage des triglycérides repose également sur ce principe en utilisant une lipase qui libère le glycérol, et une peroxydase qui réagit avec le peroxyde d'hydrogène et forme un composé rouge. Le cholestérol des HDL est dosé par un test colorimétrique enzymatique en deux phases. La première sélectionne les différentes fractions lipidiques grâce à du sulfate de dextran. La deuxième étape est similaire au dosage du cholestérol avec l'intervention d'une cholestérol-estérase et d'une cholestérol-oxydase modifiées par du polyéthylèneglycol, et d'une peroxydase qui libère un dérivé bleu-violet. Les résultats sont exprimés en mmol/l. Les coffrets nécessaires à l'ensemble de ces dosages sont fournis par RANDOX (Montpellier, France). Les LDL sont calculées à l'aide de la formule de Frieldwald à partir du cholestérol total, du cholestérol des HDL, et des triglycérides (Friedewald et al. (1972) Clin. Chem. 18 : 499-502).

Les concentrations plasmatiques d'arginine, d'ornithine et de citrulline sont déterminées sur un analyseur d'acides aminés, JEOL (Tokyo, Japon). Les acides aminés sont séparés par chromatographie échangeuse de cations. En sortie de colonne, ils sont révélés par une réaction colorée à la ninhydrine. La réaction obtenue est quantifiée par photométrie à 570 et 440 nm. Les résultats sont exprimés en µmol/l.

### 3. Etude morphologique et histologique

Le jour du sacrifice, après anesthésie, en un minimum de temps, l'animal est ouvert sur toute la longueur de la région cervico-thoracique. L'aorte est incisée le plus bas possible. A l'aide de ciseaux courbes, l'aorte est prélevée de la crosse jusqu'au niveau de la bifurcation iliaque en suivant la colonne vertébrale. L'aorte est d'abord nettoyée dans du sérum physiologique et sa tunique externe est débarrassée des tissus graisseux. Elle est fixée pendant 20 minutes dans un mélange paraformaldéhyde 2%-glutaraldéhyde 2% puis ouverte sur la longueur. Elle est ensuite replongée dans le même fixateur pendant 24h. A la suite de quoi, l'aorte est rincée dans de l'éthanol 70%, puis colorée avec du Soudan IV pendant 15 minutes. Elle est à nouveau rincée dans de l'éthanol 80% pendant 20 minutes puis sous l'eau coulante pendant 1h (22). Ensuite l'aorte est photographiée à plat avec un appareil numérique Nikon 995. Les clichés sont analysés grâce à un logiciel de planimétrie (Scion Image) qui permet de déterminer la surface des lésions. Enfin l'aorte est fixée dans le même fixateur en vue de l' étude microscopique.

Pour la microscopie optique, l'aorte est découpée en quatre segments :
- un en début et un en fin de crosse,
- un au milieu et un à la fin de l'aorte.

Des fragments adjacents sont prélevés pour la microscopie électronique.

Les prélèvements sont déshydratés progressivement dans de l'éthanol jusqu'à l'éthanol 100% puis traités par de la paraffine. Les sections ainsi imbibées de paraffine sont incluses selon une inclusion orientée qui permet d'avoir des coupes transversales. On réalise des coupes de 5 µm qui sont collées sur lames puis colorées par le HES (hémalum / érythrosine / safran du gatinais).

### 4. Analyses Statistiques

Le logiciel StatView, version 5.0, a été utilisé pour l'analyse statistique des données. Toutes les valeurs sont données comme la moyenne ± SEM. La comparaison entre les groupes est réalisée par un test de Kruskal-Wallis. Ce test est une version non paramétrique de l'analyse de variance à un facteur sur les rangs. La comparaison entre deux groupes est réalisée par un test de Mann-Whitney, qui est la version non paramétrique du test t sur séries indépendantes. On considère les valeurs significatives pour p≤0.05. La significativité des corrélations entre variables quantitatives est appréciée par le test de Spearman.

### RESULTATS

Le traitement a été effectué durant 8 semaines (de T0 début du traitement à T8).

### 1. Animaux et paramètres généraux

### 1.1.Suivi des animaux : poids et prise alimentaire

Au cours de l'expérimentation, les animaux dans les quatre groupes présentent une courbe de croissance identique (**Figure 1**). A l'arrivée à l'animalerie, les animaux du groupe témoin et du groupe arginine sont moins gros, et cette différence avec le groupe statine et le groupe statine/arginine s'est estompée dès le début du traitement (T0).

La prise alimentaire moyenne (**Figure 2**) augmente entre la période T0-T4, puis tend à être régulière jusqu'à T8. La diminution de la prise alimentaire du groupe statine après T5 est liée à la présence de deux animaux malades. De plus, deux animaux dans le groupe arginine sont morts avant la fin du protocole et n'ont pas été inclus dans l'étude.

La mesure des poids et de la prise alimentaire quotidienne reflète l'homogénéité des animaux sur ces paramètres quel que soit le groupe. Agés de 10 semaines (T4), leur poids est compris entre 1,9 et 2,1 kg ce qui correspond aux données retrouvées dans la littérature (Murakami S, Kondo Y, Sakurai T, Kitajima H, Nagate T. Taurine suppresses development of atherosclerosis in Watanabe heritable hyperlipidemic (WHHL) rabbits. Atherosclerosis. 2002 ; 163 : 79-87). La prise alimentaire augmente en fonction de l'âge (de 97,6 à 139,5g/j pour le groupe témoin par exemple) et reste relativement constante après T3.

### 1.2.Le bilan lipidique

Les traitements par l'atorvastatine et celui par l'atorvastatine plus arginine ont un effet bénéfique sur le cholestérol plasmatique (**Figure 3**) ; celui-ci diminue au cours du traitement. Cette diminution est significative pour les deux groupes versus témoin au temps T8. Le régime arginine est sans effet sur le cholestérol.

La concentration plasmatique des triglycérides (**Figure. 4**) augmente avec l'âge dans le groupe témoin et le groupe arginine. On remarque un effet positif significatif chez les animaux traités par l'atorvastatine seule (versus témoin) au T8 : la concentration en triglycérides n'augmente pas. Cet effet s'observe également pour le groupe statine/arginine (non significatif).

La concentration des HDL (**Figure 5**) dans les différents groupes évolue de la même façon que les triglycérides au cours du protocole.

De même, la concentration des LDL (**Figure 6**) suit le même tracé que celui du cholestérol. Le traitement par l'atorvastatine induit une diminution de cette concentration, de même pour le traitement atorvastatine plus arginine.

D'une part, le profil lipidique est cohérent avec la littérature : nos animaux en début d'étude (âgé de six semaines) présentent une cholestérolémie entre 20,4 mmol/l (groupe témoin) et 25,4 mmol/l (groupe statine/arginine), en accord avec le travail de Clubb & al (Clubb FJ, Cerny JL, Deferrari DA, Butler-Aucoin MM, Willerson JT, Buja LM. Development of atherosclerotic plaque with endothelial disruption in Watanabe heritable hyperlipidemic rabbit aortas. Cardiovasc Pathol 2001 ; 10 : 1-11) qui rapporte une valeur moyenne de 21,9 ± 1,5 mmol/l. D'autre part, Dowell & al (Dowell FJ, Hamilton CA, Lindop GB, Reid JL. Development and progression of atherosclerosis in aorta from heterozygous and homozygous WHHL rabbits. Effects of simvastatin treatment. Arterioscler Thromb Vasc Biol. 1995 ; 15 : 1152-60) ont effectué une étude comparative entre lapins Watanabe hétérozygotes et homozygotes et ont défini les homozygotes comme ayant un taux de cholestérol supérieur à 10 mmol/l, ce qui est bien le cas avec nos animaux.

On observe, entre T0 et T8, dans le groupe témoin et le groupe arginine une augmentation de la concentration plasmatique du cholestérol de 20% et 30% respectivement avec l'âge (**Figure 3**) alors que sous le traitement statine et statine/arginine cette concentration est diminuée de 27% pour les deux groupes. Ainsi d'après nos résultats, l'atorvastatine a un effet hypocholestérolémiant chez l'animal homozygote : elle pourrait intervenir au niveau de la synthèse hépatique du cholestérol en diminuant celle-ci. Des résultats similaires se trouvent dans la littérature mais avec des modèles différents : un travail avec la lovastatine, s'étalant sur 16 semaines chez le lapin New Zealand White (Böger RH, Bode-Böger SM, Brandes RP, Phivthong-ngam L, Böhme M, Nafe R, et al. Dietary L-Arginine reduces the progression of atherosclerosis in cholesterol-fed rabbits. Circulation 1997 ; 96 : 1282-1290) montre une diminution du cholestérol de 32%. De plus, une récente étude (Suzuki H, Kobayashi H, Sato F, Yonemitsu Y, Nakashima Y, Sueishi K. Plaque-Stabilizing Effect of Pitavastatin in Watanabe Heritable Hyperlipidemic (WHHL) Rabbits. J Atheroscler Thromb. 2003 ; 10 : 109-16) avec des lapins Watanabe sur 16 semaines, et utilisant la pitavastatine, met en avant une diminution du cholestérol plasmatique de 28,6%. La diminution de la cholestérolémie concerne principalement la fraction LDL comme le montre les résultats présentés (diminution de 27,16% pour le groupe statine et 24,5% pour le groupe statine/arginine), données que l'on retrouve aussi dans l'étude de Suzuki & al.

Au niveau des triglycérides, on observe le même profil pour le groupe témoin et le groupe arginine : les triglycérides augmentent de plus de moitié au cours du protocole (entre T0 et T8). Au contraire, les animaux sous statine (groupe statine et groupe statine/arginine) présentent une concentration quasi constante entre T0 et T8, ce qui implique un effet positif de l'atorvastatine contre l'hypertriglycéridémie.

L'arginine seule apparaît sans effet notable sur ce profil lipidique, ce qui est en accord avec de nombreux travaux (Böger RH, Bode-Böger SM, Brandes RP, Phivthong-ngam L, Böhme M, Nafe R, et al. Dietary L-Arginine reduces the progression of atherosclerosis in cholesterol-fed rabbits. Circulation 1997 ; 96 : 1282-1290 ; Brandes RP, Brandes S, Böger RH, Bode-Bôger SM, Mugge A. L-Arginine supplementation in hypercholesterolemic rabbits normalises meukocyte adhésion to non-endothelial matrix. Life Sci 2000 ; 66 : 1519-1524; Singer AH, Tsao PS, Wang BY, Bloch DA, Cooke JP. Discordant effects of dietary L-arginine on vascular structure and reactivity in hypercholesterolemic rabbits. J Cardiovasc Pharmacol 1995 ; 25 : 710-716).

### 1.3. Concentration d'arginine, d'ornithine, et de citrulline

La concentration plasmatique d'arginine (**Figure 7**) est la même pour le groupe témoin et le groupe statine. Celle-ci est doublée dans le groupe arginine à T4 (significatif *versus* témoin), mais chute à T8. L'augmentation de la concentration d'arginine dans le groupe statine/arginine est régulière mais reste très inférieure à celle observée pour le groupe arginine.

Le profil de l'ornithine (**Figure 8**) est semblable à celui de l'arginine.

La concentration plasmatique en citrulline (**Figure 9**) pour le groupe statine/arginine suit sensiblement la concentration en arginine.

D'une façon générale, la concentration en L-Arginine (ce paramètre a été dosé au temps T0, T2, T4, T6 et T8, données non montrées) a doublé dans les groupes traités, c'est-à-dire groupe arginine et groupe statine/arginine.

### 3. Etude morphologique et histologique des lésion artérielles

### 3.1. Etude macroscopique des aortes

L'évaluation de la surface des lésions (**Figure 10**) par planimétrie montre que celle-ci est plus faible sous arginine seule, et encore significativement plus faible sous arginine/statine (*versus* témoin). En revanche, on n'observe pas de différence entre le groupe témoin et le groupe traité par la statine seule. De manière surprenante l'association statine/arginine présente donc un effet synergique sur la diminution de la surface des lésions

Si l'on s'intéresse seulement à la moitié distale de l'aorte, les résultats sont très significatifs (**Figure 11**) : sous traitement atorvastatine/arginine, la surface des lésions est significativement diminuée. L'association de l'administration d'atorvastatine et d'arginine présente des effets synergiques inattendus sur la diminution des lésions athérosclérotiques car les traitements atorvastatine seule et arginine seule sont sans effet.

### 3.2. Etude microscopique de la paroi artérielle

L'observation des lames montre une moindre épaisseur des lésions pour le groupe statine/arginine en comparaison au groupe statine. Les plus grosses lésions se retrouvent dans les deux premières sections (début et à la fin de la crosse) ; sur les deux dernières coupes (milieu et fin d'aorte), les lésions sont moins fréquentes. Au niveau de leur composition, les lésions sont en majorité plus fibreuse que cellulaire (composé de macrophages spumeux et quelque polynucléaires). Il n'y a pas de tendance particulière sur la composition des lésions entre les groupes.

Au niveau macroscopique, la surface de lésion est inchangée dans le groupe statine *versus* le groupe témoin, alors que de nombreux travaux abondent dans le sens inverse, notamment celui de Kroon & al (Aliev G, Burnstock G. Watanabe rabbits with heritable hypercholesterolaemia : a model of atherosclerosis. Histol Histopathol. 1998 ; 13 : 797-817). Après administration de pravastatine à raison de 40mg/kg/j pendant neuf mois, il met en avant une diminution de l'incidence des lésions de 53 à 80%. De même, Maeso & al montrent une diminution de la taille des lésions chez des lapins NeW Zealand White traités a l'atorvastatine, 2,5mg/kg/j (Maeso R, Aragoncillo P & al Effect of Atorvastatin on endothelium-dependant constrictor factors in dyslipidemic rabbits. Gen Pharma. 2000 ; 34 : 263-272). Egalement, Böger (Böger RH, Bode-Böger SM, Brandes RP, Phivthong-ngam L, Böhme M, Nafe R, et al. Dietary L-Arginine reduces the progression of atherosclerosis in cholesterol-fed rabbits. Circulation 1997 ; 96 : 1282-1290) a montré que la lovastatine ralentissait la progression de la plaque chez le lapin New Zealand White.

Par ailleurs, le groupe arginine a une surface de lésion inférieure au témoin d'un facteur 1,3 et le traitement statine/arginine montre la surface de lésion la plus faible avec 8,8% de lésion par rapport à 13,7% pour le groupe témoin.

De plus, une corrélation inverse significative entre la surface de lésion totale et la concentration en arginine, pendant tout le protocole, est trouvée. Cette corrélation est également retrouvée pour l'ornithine.

Enfin, au niveau microscopique, il apparaît que pour le groupe statine/arginine, les lésions sont moins épaisses que pour les autres groupes.

## Revendications

1. Utilisation,
• d'au moins une statine, l'atorvastatine, ou d'un sel pharmaceutiquement acceptable de celle-ci, en association avec
• au moins un composé de formule (I) suivante : 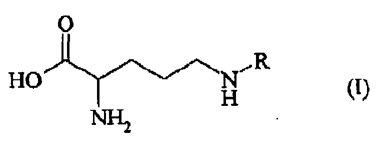 dans laquelle R représente, 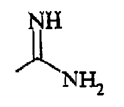 le composé de formule (I) correspondant alors à l'arginine,
ou un sel pharmaceutiquement acceptable de celui-ci,
ledit composé de formule (I) étant présent en une quantité convenant pour l'administration à un individu d'une dose de 1 g/jour à 30 g/jour, notamment de 5 g/jour à 30 g/jour pour la préparation d'un médicament destiné à la prévention ou au traitement de l'athérosclérose, notamment de l'athérosclérose primaire ou secondaire.

2. Utilisation selon la revendication 1, dans laquelle le composé de formule (I) correspond à l'isomère L.

3. Utilisation selon l'une des revendications 1 et 2, d'atorvastatine, en une quantité convenant pour l'administration à un individu d'une dose de 5 mg/j à 80 mg/j.

4. Utilisation selon la revendication 1, de L-Arginine, en une quantité convenant pour l'administration à un individu d'une dose d'au moins 10 g/jour.

5. Utilisation selon l'une quelconque des revendications 1 à 4, pour la préparation d'un médicament convenant pour une administration par voie orale.

6. Utilisation selon la revendication 5 pour la préparation d'un médicament qui se présente sous forme de poudre à diluer, de cachet, de comprimé, de gélule, ou sous toute autre forme galénique acceptable.

7. Produits contenant
• au moins une statine, l'atorvastatine, ou un sel pharmaceutiquement acceptable de celle-ci, et
• au moins un composé de formule (I) suivante : 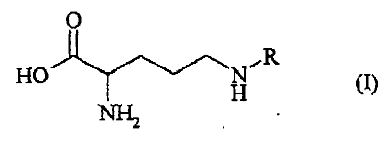 dans laquelle R représente, 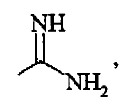 le composé de formule (I) correspondant alors à l'arginine,
ou un sel pharmaceutiquement acceptable de celui-ci,
ledit composé de formule (I) étant présent en une quantité convenant pour l'administration à un individu d'une dose de 1 g/jour à 30 g/jour, notamment de 5 g/jour à 30 g/jour comme produits de combinaison pour une utilisation simultanée, séparée ou étalée dans le temps dans le cadre du traitement de l'athérosclérose, notamment de l'athérosclérose primaire ou secondaire.

8. Produits selon la revendication 7 dans lesquels le composé de formule (I) correspond à l'isomère L.

9. Produits selon l'une des revendications 7 et 8 convenant pour l'administration à un individu d'une dose de 5 mg/j à 80 mg/j d'atorvastatine.

10. Produits selon la revendication 7 convenant pour l'administration à un individu d'une dose de L-Arginine d'au moins 10 g/jour.

## Claims

1. Use,
• of at least one statin, the atorvastatin, or of a pharmaceutically acceptable salt thereof, in combination with
• at least one compound of the following formula (I): 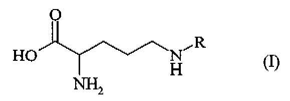 in which R represents, 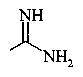 the compound of formula (I) then corresponding to the arginine,
or a pharmaceutically acceptable salt thereof,
said compound of formula (I) being present in a quantity suitable for the administration to an individual of a dose from 1 g/day to 30 g/day, in particular from 5 g/day to 30 g/day for the preparation of a medicament intended for the prevention or treatment of atherosclerosis, in particular of primary or secondary atherosclerosis.

2. Use according to claim 1, in which the compound of formula (I) corresponds to the L-isomer.

3. Use according to one of claims 1 to 2 of atorvastatin, in a quantity suitable for the administration to an individual of a dose from 5 mg/day to 80 mg/day.

4. Use according to claim 1, of L-Arginine, in a quantity suitable for the administration to an individual of a dose of at least 10 g/day.

5. Use according to any one of claims 1 to 4, for the preparation of a medicament suitable for administration by oral route.

6. Use according to claim 5, for the preparation of a medicament presented in the form of powder to be diluted, pills, tablets, capsules, or any other acceptable galenic form.

7. Products containing
• at least one statin, the atorvastatine, or a pharmaceutically acceptable salt thereof, and
• at least one compound of the following formula (I): 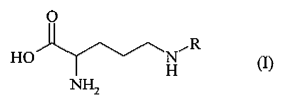 in which R represent, 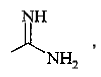 the compound of formula (I) then corresponding to the arginine,
or a pharmaceutically acceptable salt thereof,
said compound of formula (I) being present in a quantity suitable for the administration to an individual of a dose from 1 g/day to 30 g/day, in particular from 5 g/day to 30 g/day as combination products for simultaneous or separate use or use spread over time within the framework of the treatment of atherosclerosis, in particular of primary or secondary atherosclerosis.

8. Products according to claim 7, in which the compound of formula (I) corresponds to the L-isomer.

9. Products according to one of claims 7 to 8, suitable for the administration to an individual of a dose from 5 mg/day to 80 mg/day of atorvastatin.

10. Products according to claim 7, suitable for the administration to an individual of a dose of L-Arginine of at least about 10 g/day.

## Patentansprüche

1. Verwendung
• mindestens eines Statins, von Atorvastatin oder eines pharmazeutisch verträglichen Salzes davon, in Assoziation mit
• mindestens einer Verbindung mit der folgenden Formel (I): 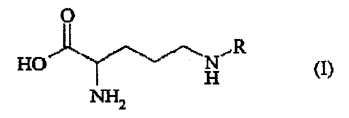 worin R für 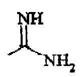 steht, wobei die Verbindung mit der Formel (I) dann Arginin entspricht,
oder einem pharmazeutisch verträglichen Salz davon,
wobei die Verbindung mit der Formel (I) in einer Menge, die sich für die verabreichung an ein Individuum eignet, in einer Dosis von 1 g/Tag bis 30 g/Tag, insbesondere 5 g/Tag bis 30 g/Tag vorhanden ist,
zur Herstellung eines Medikaments, das zur Vorbeugung oder Behandlung von Atherosklerose, insbesondere primärer oder sekundärer Atherosklerose, bestimmt ist.

2. Verwendung nach Anspruch 1, wobei die Verbindung mit der Formel (I) dem Isomer L entspricht.

3. Verwendung von Atorvastatin nach einem der Ansprüche 1 bis 2 in einer Menge, die sich für die Verabreichung an ein Individuum eignet, in einer Dosis von 5 mg/T bis 80 mg/T.

4. Verwendung von L-Arginin nach Anspruch 1 in einer Menge, die sich für die Verabreichung an ein Individuum eignet, in einer Dosis von mindestens 10 g/Tag.

5. Verwendung nach irgendeinem der Ansprüche 1 bis 4 zur Herstellung eines Medikaments, das sich für die orale Verabreichung eignet.

6. Verwendung nach Anspruch 5 zur Herstellung eines Medikaments, das die Form eines Pulvers zum Auflösen, einer Oblatenkapsel, einer Tablette, einer Gelatinekapsel oder jeder anderen verträglichen galenischen Form aufweist.

7. Produkte, enthaltend
• mindestens ein Statin, Atorvastatin oder ein pharmazeutisch verträgliches Salz davon, und
• mindestens eine Verbindung mit der folgenden Formel (I): 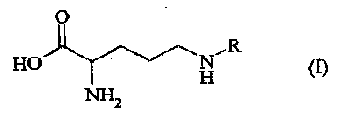 worin R für 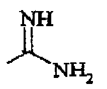 steht, wobei die Verbindung mit der Formel (I) dann Arginin
oder einem pharmazeutisch verträglichen Salz davon entspricht,
wobei die Verbindung mit der Formel (I) in einer Menge, die sich für die Verabreichung an ein Individuum eignet, in einer Dosis von 1 g/Tag bis 30 g/Tag, insbesondere 5 g/Tag bis 30 g/Tag vorhanden ist,
als Kombinationsprodukte zur gleichzeitigen, getrennten oder zeitlich gestaffelten Verwendung im Rahmen der Behandlung von Atherosklerose, insbesondere von primärer oder sekundärer Atherosklerose.

8. Produkte nach Anspruch 7, wobei die Verbindung mit der Formel (I) dem Isomer L entspricht.

9. Produkte nach einem der Ansprüche 7 und 8, die für die Verabreichung an ein Individuum geeignet sind, in einer Dosis von 5 mg/T bis 80 mg/T Atorvastatin.

10. Produkte nach Anspruch 7, die sich für die Verabreichung an ein Individuum eignen, einer L-Arginin-Dosis von mindestens 10 g/Tag.
